# EUROPEAN PATENT SPECIFICATION

(11) **EP 3 831 232 B1**
(45) Date of publication and mention of the grant of the patent: **03.07.2024**
(21) Application number: 19844278.2
(22) Date of filing: 05.02.2019
(51) Int. Cl.: A41D 13/11, A61K 9/70, A61K 41/00, A61K 47/32, A61P 31/00, A61P 31/04, A61P 31/12, A61P 37/08, A62B 18/02, B32B 3/08, B32B 3/28, B32B 5/02, B32B 5/26, B32B 7/04, B32B 25/10, B32B 27/12, B32B 27/18, D04H 3/005

(54) **MASK**
MASKE
MASQUE

(30) Priority: 02.08.2018 JP 2018145831
(43) Date of publication of application: 09.06.2021
(73) Proprietor: Protecart Co., Ltd., Yokkaichi-shi, Mie, 512-0935 (JP)
(72) Inventor: ITO, Yoshiaki, Yokkaichi-shi, Mie 512-0935 (JP)
(74) Representative: Grünecker Patent- und Rechtsanwälte PartG mbB
(86) International application number: PCT/JP2019/004070
(87) International publication number: WO 2020/026472

(56) References cited:
- CN-A- 106 388 081
- JP-A- 2006 087 655
- JP-A- 2011 011 004
- JP-A- 2014 110 820
- KR-Y1- 200 315 157

## Description

### FIELD

The present invention relates to a mask.

### BACKGROUND

Respiratory infections such as influenza develops by viruses, bacteria or the like invading into the respiratory tract. In order to prevent the invasion of the aforementioned viruses, bacteria or the like into the respiratory tract as well as preventing the spread of droplets caused by coughing or sneezing, the use of masks is recommended. In recent years, masks have also been used as a countermeasure against pollen and PM2.5.

Here, a mask is configured of a body portion that is air-permeable and which covers at least one portion (mainly the nose and mouth) of a face of a wearer, and a fixing portion that fixes the body portion against the face of the wearer. A conventional mask commonly uses nonwoven fabric or woven fabric for the body portion. While the air-permeability of the nonwoven fabric or woven fabric secures respiration of the wearer, the mask filters particulate matters represented by pollen and PM2.5, as well as droplet viruses, bacteria or the like by its function as a filter (for example, JP2014-198165A). This accordingly prevents the particulate matters, droplet viruses, bacteria or the like from invading into the respiratory tract, as well as spreading to the surroundings. Masks that include a photocatalyst for purification and sterilization are also known (for example, CN106388081A and KR200315157Y1). JP 2011-011004 A proposes to easily prevent a person from sucking bacteria and unnecessary gas, and discloses a mask that uses an air cleaning device composed of an α-ray radiation part and a carbon containing part. The α-ray radiation part uses an air cleaning device composed of a device in which an oxide of an α-ray generation source is arranged in a network medium. JP 2014-110820 A and JP 2006-087655 A disclose masks according to the preamble of claim 1.

### [Citation List]

### [Patent Document]

[Patent Document 1] JP2014-198165A (Page 5)

### SUMMARY

### [Problems to be Solved by the Invention]

However, although the mask described in the aforementioned Patent Document 1 is capable of filtering particulate matters, droplet viruses, bacteria or the like (hereinafter, collectively referred to as viruses or the like) at the body portion, the filtered viruses or the like remain active. As a result, if the viruses or the like once filtered at the body portion disperses to the surroundings due to some kind of cause, there was the fear that the viruses or the like would invade into the respiratory tract of the surrounding people or the wearer of the mask and cause the onset of infections, pollen allergy or the like.

The present invention is accomplished to resolve the above conventional problems, and an object thereof is to provide a mask that inactivates viruses or the like filtered at the body portion as well as the surrounding viruses or the like by comprising a layer in the body portion that generates a weak radiation, and having a particularly remarkable effect in preventing infections and pollen allergy.

### [Means for Solving the Problems]

In order to achieve the above object, a mask according to the present invention is defined in independent claim 1. Preferred embodiments are set out in the dependent claims.

### [Advantageous Effects of Invention]

According to the mask of the present invention having the above configurations, water molecules present in the surrounding air become ionized or excited by the weak radiation generated around the body portion. This generates hydroxyl radicals, and further from the hydroxyl radicals, hydrogen peroxide is also generated. The generated hydroxyl radicals and hydrogen peroxide enable inactivation of the viruses, bacteria or the like filtered at the body portion or that are present around the body portion. In particular, it is possible to inactivate bacteria including *Staphylococcus aureus, Pseudomonas aeruginosa, Salmonella enteritidis, Escherichia coli* (0-157), and *Moraxella osloensis,* and viruses with envelopes, for example Influenza virus (e.g., Influenza A virus, avian influenza). Furthermore, the weak radiation generated from the body portion has an effect of stabilizing the immune system of the wearer and improving their immune functions by the so-called hormesis effect. As a result, the ability to resist pollen allergy, infections or the like improve, and by combining this with the inactivation of the above viruses or the like, an extremely large effect can be expected against pollen allergy and infections or the like.
On the other hand, the weak radiation generated from the body portion also has an effect of reducing various components that serve as a cause for bad odor, such as ammonia gas, acetic acid gas, trimethylamine gas, isovaleric acid gas, nonenal gas, and indole gas. Therefore, the displeasure feeling for the wearer of the mask may be reduced in a case of staying in a space with bad odor. Moreover, it is also possible to prevent the mask from being contaminated by the bad odor component that the wearer themselves give out from inside their mouth.

### BRIEF DESCRIPTION OF DRAWINGS

Fig. 1 is an external view of a mask according to the present embodiment.
Fig. 2 is a cross sectional view of a body portion of the mask according to the present embodiment that is cut in a vertical direction.
Fig. 3 is a view describing the operation and effect of the mask according to the present embodiment.

### BRIEF DESCRIPTION OF TABLES

Table 1 is a test result of a filtration effect test against particulate matters.
Table 2 is a test result of a filtration effect test against bacteria and viruses.
Table 3 is a test result of a filtration effect test against pollen.
Table 4 is a test result of an inactivation test against Influenza A virus (after 30 minutes).
Table 5 is a test result of an inactivation test against Influenza A virus (after 2 hours).
Table 6 is a test result of an inactivation test against bacteria.
Table 7 is a test result of an inactivation test against bacteria.
Table 8 is a test result of an inactivation test against bacteria.
Table 9 is a view illustrating a theoretical reduction rate of viruses and bacteria.
Table 10 is a result of a preliminary test of an inactivation test against avian influenza.
Table 11 is a result of a final test of the inactivation test against avian influenza.
Table 12 is a view depicting reduction rates of various gases that serve as bad odor components.

### DESCRIPTION OF EMBODIMENTS

The following describes in details of the mask according to the present invention based on one specified embodiment, with reference to the drawings. In particular, the following description describes an example that applies the mask according to the present invention as a sanitary mask (face mask). However, the present invention is not necessarily only applied to sanitary masks, and is also applicable to surgical masks and dust-proof masks.

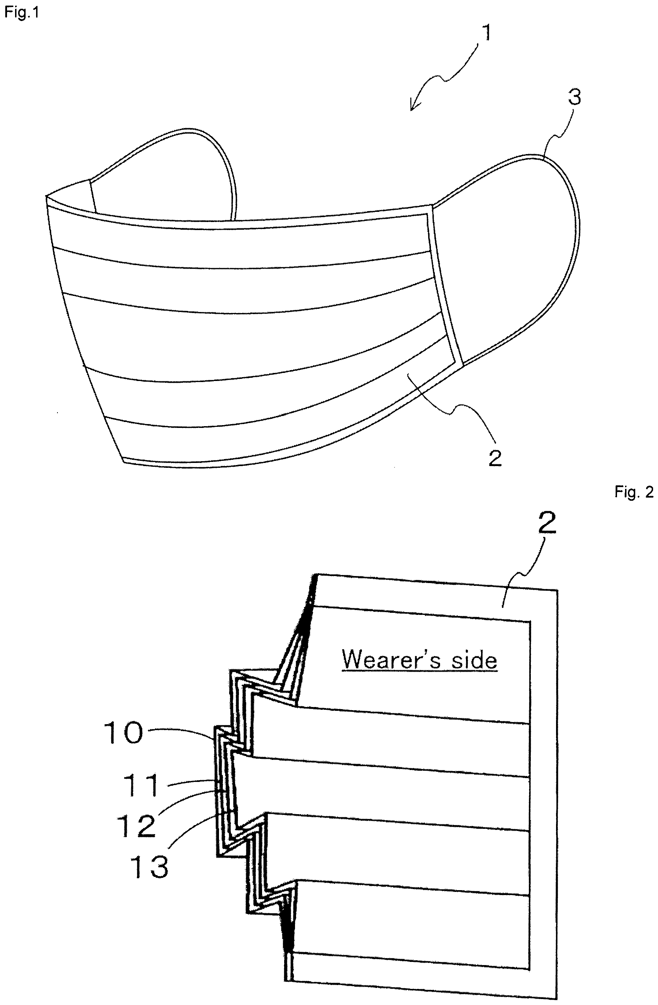

First, a schematic configuration of a mask 1 according to the present embodiment will be described, with reference to Fig. 1. Fig. 1 is an external view of the mask 1 according to the present embodiment.

As illustrated in Fig. 1, the mask 1 according to the present embodiment includes a body portion 2 that covers one portion, more specifically the nose and mouth of a face of a wearer, and a fixing portion 3 for fixing the body portion 2 against the face of the wearer.

The body portion 2 uses material having air-permeability such as nonwoven or woven fabric, and even in a state in which the body portion 2 is closely adhered to the wearer, respiration of the wearer is secured. On the other hand, these nonwoven and woven fabrics also have the function as a filter, and filters particulate matters represented by pollen and PM2.5, as well as droplet viruses and bacteria. It is desirable for the body portion 2 to have a multilayer structure for improving the filtration function and durability. Particularly in the present embodiment, among the multilayer structure, at least one layer is to be a radiation generating layer that generates a weak radiation (or electromagnetic wave) to its surroundings as later described. However, the multilayer structure of the body portion 2 is not essential, and a single layer structure with just the radiation generating layer is also possible.

Moreover, the mask 1 depicted in Fig. 1 is a pleated-type mask processed with pleats in a horizontal direction on the body portion 2, however the pleating is not essential, and may be a dimensionally-formed mask having a dimensional shape that fits with the shape of the face of the wearer.

On the other hand, the fixing portion 3 is coupled to the body portion 2 at the left and right edges of the body portion 2. The fixing portion 3 is a string form member, and connects an upper end and a lower end in a ring form at the left and right edges of the body portion 2. For example, an elastic rubber string or elastic nonwoven fabric or the like is used. By putting the fixing portion 3 on both ears of the wearer, the mask 1 is worn on the face of the wearer.

Next describes in detail of the structure of the body portion 2 of the mask 1. Fig. 2 is a cross sectional view of the body portion 2 of the mask 1 according to the present embodiment that is cut in a vertical direction.

As illustrated in Fig. 2, in the mask 1 of the present embodiment, the body portion 2 is of a four-layered structure. The body portion 2 includes a first layer 10 serving as the outermost layer when the wearer wears the mask, a second layer 11 disposed on an inner side of the first layer 10, a third layer 12 that is further disposed on an inner side of the second layer 11, and a fourth layer 13 disposed as the innermost layer (namely, the layer in contact with the face of the wearer). Peripheral portions of each layer are welded to configure the body portion 2. However, the number of layers may vary as appropriate, and may be three layers or less or five layers or more.

In the present embodiment, of the first layer 10, second layer 11, third layer 12, and fourth layer 13, particularly the second layer 11 is to be the radiation generating layer that generates weak radiation to the surroundings. The remaining first layer 10, third layer 12, and fourth layer 13 are not particularly limited, and any nonwoven or woven fabric may be used that is made of natural fiber, synthetic fiber, or a mixture of these fibers. The natural fiber may be any one of plant fibers such as cotton or hemp, or animal fibers such as wool or silk. On the other hand, examples of the synthetic fiber include polyamide, polyester, polyolefin, polyacronitrile, and polyurethane fibers, however the polyolefin or polyester fibers are preferred. In particular, the present embodiment is to be a nonwoven fabric made of polypropylene fibers. Particularly for the third layer 12 that is positioned inside, it is desirable to use a nonwoven fabric that has a high performance filter function with a maximum pore size smaller than the other layers. For example, the nonwoven fabric of the third layer 12 is to be a nonwoven fabric having a blocking rate (filtration efficiency) of 99% or more in each test of VFE, BFE, PFE, and pollen filtration. Test methods and conditions of the pollen filtration test are to be the conditions and method disclosed in Table 3 later described.

On the other hand, the second layer 11 being the radiation generating layer is to be a nonwoven fabric having a radiation generating substance applied on at least one plane thereof. The radiation generating substance is a substance that causes generation of a weak radiation to its surroundings, and many rare earth ore based substances shall be subjected to this. For example, thorium oxide is one example. Thorium oxide and ceramics containing zircon, zirconia, alumina, silica or the like that emits far-infrared rays are crushed and mixed, and the crushed mixture crushed to an extremely minute diameter (for example, 0.8 µm or less) is mixed with a binder and applied on the nonwoven fabric, to manufacture the second layer 11. In an alternative that is not claimed, the second layer 11 is manufactured by blending the aforementioned crushed mixture into the raw materials of the nonwoven fabric and then manufacturing the nonwoven fabric from those raw materials.

Examples of the nonwoven fabric used as the second layer 11 include polyamide, polyester, polyolefin, polyacronitrile, polyurethane fiber or the like, however polyolefin or polyester fiber is preferred. Since it is necessary to weld with other layers, it is preferable to make the nonwoven fabric the same type as the other layers. Particularly in the present embodiment, the nonwoven fabric is to be made of polypropylene fibers capable of welding at a low temperature (around 160 °C) when welding the layers. Moreover, the radiation generating layer may be one in which the radiation generating substance is applied not on nonwoven fabric but on woven fabric. Moreover, as for the nonwoven fabric used for the second layer 11, in order to secure sufficient air-permeability in a state in which the radiation generating substance is applied thereon, it is desirable to use a nonwoven fabric thinner than a nonwoven fabric used in common masks. More specifically, its mass per unit area is to be not more than 17 g/m². Moreover, the mass per unit area in the state in which the radiation generating substance is applied thereon is to be not more than 32 g/m², more preferably not more than 27 g/m².

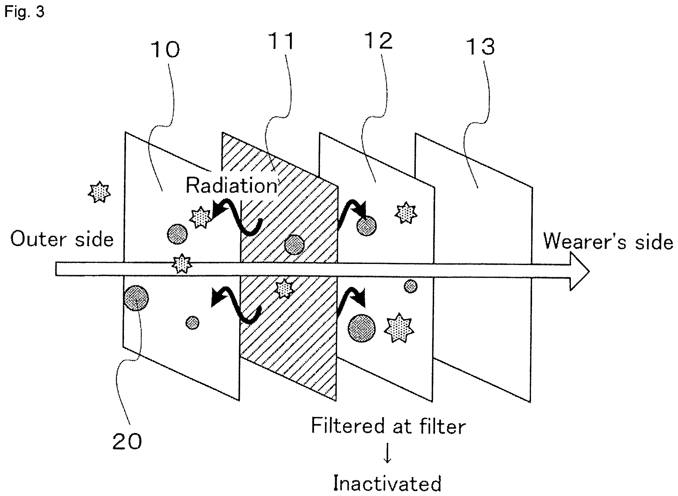

Next describes the operation and effect of the mask 1 having the aforementioned radiation generating layer, with reference to Fig. 3. Fig. 3 is a view describing the operation and effect of the mask 1 according to the present embodiment.

As illustrated in Fig. 3, when particulate matter represented by pollen and PM2.5, droplet viruses, bacteria or the like (hereinafter, these collectively referred to as filtered object 20) enters into the body portion 2 together with outside air from its outer side, the filtered object 20 is filtered at any one of the first layer 10 to fourth layer 13. In particular, the third layer 12 has a high filtering function as described above, and thus filters the filtered object 20 more securely.

Here, the second layer 11 being the radiation generating layer generates a weak radiation to its surroundings. Energy of the generated radiation is absorbed by water molecules contained in ambient air, and the water molecules ionize and become excited. As a result, water molecule ions (H₂O⁺) and excited water molecules (H₂O*) are generated, as in the following formulae (1) and (2):

H₂O + radiation → H₂O⁺ + e⁻ ... (1)

H₂O + radiation → H₂O* ... (2)

Moreover, the ions of the water molecules (H₂O⁺) are extremely unstable, and thus are decomposed as in the following formula (3) and generate hydroxyl radicals and H₃O⁺.

H₂O⁺ + H₂O → H₃O⁺ + OH radical ... (3)

Furthermore, the excited water molecules (H₂O*) cleave, which generate hydroxyl radicals and hydrogen radicals, as in the following formula (4):

H₂O* → OH radical + H radical ... (4)

Moreover, the electron (e⁻) that has jumped out from the molecule is captured between other water molecules, which thus generates aqueous electrons (e^{aq-}).

Furthermore, by the radicals bonding again, hydrogen molecules and hydrogen peroxide are generated, as in the following formulae (5) and (6):

OH radical + OH radical → H₂O₂... (5)

H radical + H radical → H₂ ... (6)

As described above, the radiation generated from the second layer 11 generates radicals such as a hydroxyl radicals, hydrogen radicals or the like, molecular products such as H₂, HzOz or the like, and aqueous electrons (e^{aq-}), as a result of reacting with water molecules contained in the air.

Moreover, the hydroxyl radicals generated by the water molecules being ionized and excited inactivate the filtered object 20. For example, HzOz having high antibacterial power is generated as shown in the aforementioned formula (5), thus inactivating the filtered object 20.

The inactivation effect is present for 1 m to within 2 m around the second layer 11, and thus it is possible to securely inactivate the filtered object 20 filtered at the first layer 10 to fourth layer 13.

Fig. 3 illustrates an example in which the filtered object 20 enters together with the outside air to the wearer's side from the outside; the same effect may be expected in a case of the filtered object 20 entering from the wearer's side to the outer side by coughs and sneezes by the wearer.

Furthermore, the weak radiation generated by the second layer 11 being the radiation generating layer has an effect of stabilizing the immune system of the wearer and improving their immune functions by the so-called hormesis effect. As a result, the ability to resist pollen allergy, infections or the like improve, and by combining this with the inactivation of the filtered object 20, an extremely large effect may be expected against pollen allergy and infections or the like.

On the other hand, the hydroxyl radicals generated from the water molecules also have effect in deodorization as depicted in the following formula (7):

NH₃ + OH radical → N₂ + H₂O ... (7)

Furthermore, the hydroxyl radicals have effect in reducing various components that serve as a cause of bad odor other than ammonia gas, for example acetic acid gas, trimethylamine gas, isovaleric acid gas, nonenal gas, indole gas or the like. Therefore, the displeasure feeling for the wearer of the mask 1 may be reduced in a case of staying in a space with bad odor. Moreover, it is also possible to prevent the mask 1 from being contaminated by the bad odor component the wearer themselves give out from inside their mouth.

### [Examples]

The following describes Examples of the present invention, in comparison with Comparative Example.

### (Example)

The mask 1 was manufactured by having the first layer 10 be spunbond nonwoven fabric (20 g/m²), the second layer 11 be nonwoven fabric in which a radiation generating substance of 10 g/m² is applied on nonwoven fabric of polypropylene fiber (17 g/m²), the third layer 12 be meltblown nonwoven fabric (22 g/m²), and the fourth layer 13 be spunbond nonwoven fabric (20 g/m²), and welding the peripheral parts of layers. Then, the following tests were performed to the manufactured mask 1.

### (Filtration effect test against particulate matter)

Pearl-shaped polystyrene standard particles having a particle size of 0.1 µm were penetrated into the body portion 2 from the first layer 10 side at 28.3 L/min. The detailed testing method is depicted in Table 1. Further, filtration results (filtration efficiency) of the pearl-shaped polystyrene standard particles are depicted in Table 1.

The test was performed for a total of five times as depicted in Table 1, and in each test the pearl-shaped polystyrene standard particles demonstrated a filtration efficiency of not less than 99.4%, and their average were also 99.6%. Namely, this demonstrates that the mask of the Example has extremely high filtration effect against particulate matter.

### (Filtration effect test against bacteria and viruses)

BFE test against bacteria (test bacterium: *Staphylococcus aureus*) and VFE test against viruses (test virus: Bacteriophage, host bacterium: *Escherichia coli*) were performed. The detailed testing method is depicted in Table 2. Further, filtration results (filtration efficiency) of each test are depicted in Table 2.

The test was performed three times each (total six times) as depicted in Table 2, and the BFE test demonstrated a filtration efficiency of not less than 99.0%, and the average was also 99.4%. Moreover, the VFE test demonstrated a filtration efficiency of not less than 99.2%, and the average was also 99.5%. Namely, this demonstrates that the mask of the Example has an extremely high filtration effect against bacteria and viruses.

### (Filtration effect test against pollen)

Lycopodium powder as replacement particles of pollen were penetrated from the first layer 10 side to the body portion 2 at a flow rate of 28.3 L/min. The detailed testing method is depicted in Table 3. Further, filtration results (filtration efficiency) of the replacement particles of pollen are depicted in Table 3.

The test was performed for a total of three times as depicted in Table 3, and in each test the replacement particles of pollen demonstrated a filtration efficiency of not less than 99.4%, and their average was also 99.8%. Namely, this demonstrates that the mask of the Example has an extremely high filtration effect against pollen.

### (Inactivation test against Influenza A virus)

In the following test, "nonwoven fabric in which a radiation generating substance of 10 g/m² is applied on a nonwoven fabric of polypropylene fiber (17 g/m²)" that may be used as the second layer 11 of the mask is used as the Example, and "woven fabric of 100% cotton" is used as the Comparative Example. A virus fluid of Influenza A virus was inoculated to the Example and Comparative Example as a testing virus fluid. Then, antiviral activity values were detected 30 minutes later and two hours later. The detailed testing method and test results are depicted in Table 4 and 5. The result of after 30 minutes is depicted in Table 4, and the result of after 2 hours is depicted in Table 5.

As depicted in Table 4, an antiviral activity value Mv(log(Va)-log(Vc)) detected 30 minutes later was 0.7. On the other hand, as depicted in Table 5, an antiviral activity value Mv(log(Va)-log(Vc)) detected 2 hours later was 3.7. Moreover, Table 9 depicts a theoretical reduction rate of the number of viruses calculated from the values of log(Va) and log(Vc). As for the antiviral activity value Mv, it is generally determined as having a high effect in the inactivation of viruses (reduction in the number of viruses) if it is not less than 2.0, and is determined as having a remarkably high effect if it is not less than 3.0. Although the value is lower at the time of 30 minutes later than that of 2 hours later, the number of viruses is reduced by nearly 80% as depicted in Table 9, and is definitely being reduced. The antiviral activity value at 2 hours later demonstrates an extremely high value of 3.7, and the theoretical reduction rate of viruses became 99.98%. Namely, this demonstrates that the mask of the Example has an extremely high effect in the inactivation of Influenza A virus.

### (Inactivation test 1 against bacteria)

*Staphylococcus aureus, Pseudomonas aeruginosa,* and *Escherichia coli* 0-157, added with a surfactant (Tween80), were inoculated to the Example and Comparative Example as test bacterial suspensions. Thereafter, antibacterial activity values at 18 hours later were detected for each bacteria. The detailed testing method and test results are depicted in Table 6.

As depicted in Table 6, the antibacterial activity values detected 18 hours later were 5.8 for *Staphylococcus aureus,* 6.0 for *Pseudomonas aeruginosa,* and 5.0 for *Escherichia coli* 0-157. Moreover, Table 9 depicts the theoretical reduction rate of the number of bacteria calculated from the common logarithm values of viable bacteria count. As for the antibacterial activity value, it is generally determined as having a high effect in inactivation of bacteria if this is not less than 2.0, and is determined as having a remarkably high effect if this is not less than 3.0. Each of the antibacterial activity values at 18 hours later demonstrates a remarkably high value of 5.0 or more, and the theoretical reduction rate of bacteria each was not less than 99%. Namely, this demonstrates that the mask of the Example has an extremely high effect in the inactivation of the aforementioned bacteria.

### (Inactivation test 2 against bacteria)

*Moraxella osloensis* and *Salmonella enteritidis,* added with a surfactant (Tween80), were inoculated to the Example and Comparative Example as test bacterial suspensions. Thereafter, antibacterial activity values at 18 hours later were detected for each bacteria. The detailed testing method and test results are depicted in Tables 7 and 8.

As depicted in Tables 7 and 8, the antibacterial activity values detected 18 hours later were 6.0 for *Moraxella osloensis,* and 3.3 for *Salmonella enteritidis.* Moreover, Table 9 depicts the theoretical reduction rate of the number of bacteria calculated from the common logarithm values of viable bacteria count. As for the antibacterial activity value, it is generally determined as having a high effect in inactivation of bacteria if this is not less than 2.0, and is determined as having a remarkably high effect if this is not less than 3.0. Each of the antibacterial activity values at 18 hours later demonstrates a remarkably high value of not less than 3.0, and particularly with *Moraxella osloensis,* the theoretical reduction rate of bacteria was not less than 99.9%. Although Salmonella enteritidis was lower in reduction rate than *Moraxella osloensis,* the virus was reduced by 60% or more. Namely, this demonstrates that the mask of the Example has an extremely high effect in the inactivation of the aforementioned bacteria.

### (Inactivation test against avian influenza)

The following test tests the inactivation effect against avian influenza in particular. As Example 1 in the present test, "nonwoven fabric in which a radiation generating substance of 10 g/m² is applied on nonwoven fabric having a mass per unit area of 17 g/m²" is used. As Example 2, "nonwoven fabric in which a radiation generating substance of 15 g/m² is applied on nonwoven fabric having a mass per unit area of 17 g/m²" is used. As Example 3, which does not conform to the invention, "nonwoven fabric in which a radiation generating substance of 10 g/m² is applied on nonwoven fabric having a mass per unit area of 50 g/m²" is used. Each nonwoven fabric is a member that may configure the second layer 11 of the mask 1. Moreover, "woven fabric (standard fabric) of 100% cotton" is used as the Comparative Example.

### [Used Virus]

Avian influenza (A/Cygnus columbianus bewickii/Shimane/499/83(H5N3) strain was used.

This virus was inoculated into the allantoic cavity of an embryonated egg of 10 days old and cultured for 2 days at 35 °C then the allantoic fluid was collected to use as the virus fluid. The virus fluid used for the present test was used in the test upon calculating its 50% embryo infectious dose (EID₅₀). Moreover, the present virus strain is a low pathogenic avian influenza isolated by Otsuki et al. from feces of Cygnus columbianus that came flying into Shimane prefecture in 1983, and has been confirmed that the high pathogenicity is acquired by passage from generation to generation through chicks.

### [Used egg]

An SPF fertilized egg was incubated, and was used for the test at 10 days old.

### [Preliminary test details]

In order to confirm that no virus inactivation effect remains within a washing fluid based on SCDLP culture from the nonwoven fabric, the following control test was performed.
(1) Each of the nonwoven fabrics and standard fabric of Examples 1 and 3 and Comparative Example was cut into 1.5 cm squares; 0.2 g was stacked for each and were placed in a zippered bag made of polyethylene (note: no sterilization of samples was performed by an autoclave or the like before the test).
(2) Each of the test object was impregnated into 2 mL of SCDLP culture, was rubbed well from the outside of the polyethylene bag, then the fluid was squeezed out and was collected to use as the washing fluid.
(3) 900 µL of each washing fluid was poured into respective test tubes.
(4) 100 µL of the virus fluid (10^{7.5}EID₅₀/0.2mL) was added into each test tube, was left at room temperature for 30 minutes and then virus titer of the mixed fluid was measured, to compare the residual virus titers.

### [Preliminary test results]

Table 10 depicts the test results. As a comparison target, the residual virus titer of the virus fluid after leaving for 30 minutes at room temperature is also depicted. As depicted in Table 10, it can be seen that the residual virus titer of each test tube has not greatly decreased, with respect to the residual virus titer of the virus fluid. Namely, no inactivation effect against avian influenza was observed in the washing fluid by SCDLP culture in any case. As from the above, it was confirmed that the SCDLP culture may be used as the washing fluid for the present test.

### [Final test details]

The inactivation effect against avian influenza is tested with the nonwoven fabrics and standard fabric of Examples 1 to 3 and Comparative Example.
(1) Each of the nonwoven fabrics and standard fabric was cut into 1.5 cm squares; 0.2 g was stacked for each and were placed in a zippered bag made of polyethylene (note: no sterilization of samples are performed by an autoclave or the like before the test).
(2) The virus fluid was diluted in phosphate buffered saline (PBS), to prepare the virus titer to approximately 10^{7.5}EID₅₀/0.2 mL.
(3) Each of the test object was impregnated into 200 µL of the virus fluid, and was left to react for 2 hours at room temperature according to the rules, and in addition for 1 hour, 30 minutes, and 10 minutes.
(4) After the reaction, 1.8 mL of SCDLP culture was added, to terminate the reaction.
(5) The test objects were rubbed well from outside the polyethylene bag, the virus fluid was squeezed and collected, and further was diluted by 10 times with PBS.
(6) The fluid was inoculated into allantoic cavities of three fertilized eggs 10 days old 0.2 mL each per dilution stages, and these were cultured for 3 days at 35 °C.
(7) After culturing, the fluid was cooled for one night, then the allantoic fluid was collected to react with 0.5% chicken red blood cell suspension. Whether the virus multiplied or not was determined by the result of agglutination. As for the residual virus titer, EID₅₀ was calculated by the Reed and Muench method.
(8) The residual virus titer of the test virus was found by leaving the virus fluid at room temperature for 2 hours, 1 hour, 30 minutes, and 10 minutes, respectively, then a 10 fold dilution in SCDLP culture, followed by a 10 fold serial dilution in PBS.

### [Final test results]

Table 11 depicts the test results. In order to confirm the reproducibility, the test was carried out twice under the same conditions. Since the nonwoven fabric was water-repellent and its water absorbency was not good, at the time of reaction the test nonwoven fabric was thoroughly rubbed after dropping the virus fluid thereon, to blend in the virus fluid.

The nonwoven fabric of Example 1 (10 g/m² of radiation generating substance applied on an nonwoven fabric having a mass per unit area of 17 g/m²) demonstrated a reduction in the residual virus titer to approximately 1/100000, in the case of reaction for 2 hours. Moreover, even in the cases in which the reaction times were reduced to 10 minutes, 30 minutes, or 1 hour, it was confirmed that the residual virus titer was reduced to approximately 1/10 to 1/100.
Moreover, it was also confirmed that the nonwoven fabric of Example 2 (15 g/m² of radiation generating substance applied on an nonwoven fabric having a mass per unit area of 17 g/m²) demonstrated a reduction in the residual virus titer to approximately 1/100000 even in the cases in which the reaction times were shortened to 30 minutes or 1 hour compared to Example 1. Moreover, even in the case in which the reaction time was shortened to 10 minutes, it was confirmed that the residual virus titer was reduced to not more than approximately 1/10000.
Moreover, the nonwoven fabric used in Example 3 (10 g/m² of radiation generating substance applied on an nonwoven fabric having a mass per unit area of 50 g/m²), although demonstrated a smaller inactivation effect compared to Examples 1 and 2, a slight reduction effect was confirmed when compared with the standard fabric used in Comparative Example (woven fabric of 100% cotton).

Contemplating the above results, it is considered that the mask 1 according to the present embodiment achieves an inactivation effect against avian influenza. Comparing Examples 1 to 3, it is considered that the easiness in making the radiation generating substance applied on the nonwoven fabric be in contact with the virus fluid is largely involved in the inactivation effect. Namely, a nonwoven fabric with a rough mass per unit area and being a nonwoven fabric having a high application density of the radiation generating substance enables easy contact of the radiation generating substance with the virus fluid, and thus is considered to demonstrate a higher inactivation effect. For example, a nonwoven fabric with a mass per unit area of not more than 17 g/m² demonstrates a high inactivation effect. Moreover, the application density of the radiation generating substance of 10 g/m² or more demonstrates a high inactivation effect. However, if the radiation generating substance is too high in application density, the air-permeability decreases and the functionality as a mask decreases. More specifically, the mass per unit area of the nonwoven in the state on which the radiation generating substance is applied is desirably not more than 32 g/m², more preferably not more than 27 g/m². Therefore, as for the mass per unit area of the nonwoven fabric and the application density of the radiation generating substance, it is a matter of course to consider the aforementioned inactivation effect against avian influenza, but is it also necessary to select as appropriate in consideration of other functions as a mask.

The avian influenza virus is originally a virus to which birds such as waterfowls and chickens are infected, and is extremely low in possibility that humans are infected thereto. Moreover, even if a human is infected, it is extremely low in possibility that the virus will be infected from human to human. However, there is the potential for the virus to become a virus adapted to humans (namely, a virus to which humans become infected) by gene mutation or by genetic hybridization. Compared to other influenza viruses, mostly none of humans are immune to the avian influenza virus; therefore, there is the fear that in a case in which the virus becomes one adapted to humans (novel influenza virus), this may cause a pandemic. Once this causes a pandemic, it is not easy for the virus to be eradicated, and an immense damage is anticipated worldwide. Therefore, it is considered an important issue worldwide to erect a measure to prevent the pandemic from occurring due to the avian influenza virus.

As an effective measure to prevent the occurrence of a pandemic by the avian influenza virus, the prevention of infection from bird to human or from human to human may be mentioned. By being able to prevent the infection from birds to humans, it would be possible to destroy the possibility that the avian influenza virus become a virus adapted to humans. Moreover, by being able to prevent the infection from human to human, it would be possible to prevent the spread of infection even if the avian influenza virus adapts to humans.

Referring to the aforementioned results of the inactivation test against the avian influenza virus, the mask according to the present embodiment achieves an inactivation effect against the avian influenza virus. Therefore, by wearing the mask, it can be expected that infections of the avian influenza virus from birds to humans, as well as from human to human are prevented. Namely, the mask according to the present embodiment may serve as an effective measure for preventing the occurrence of a pandemic caused by the avian influenza virus.

Moreover, by using the nonwoven fabric of Examples 1 to 3 at locations that may serve as a cause for the spread of the avian influenza virus between birds, such as a poultry farm for example, it is also possible to expect the prevention of the infection of the avian influenza virus between birds. For example, a method of arranging the nonwoven fabric of Examples 1 to 3 on the floor or wallpaper of the poultry farm may be considered.

In the aforementioned Example, tests were conducted to confirm the inactivation effect, particularly for Influenza A virus and avian influenza; a similar high effect can be expected for viruses with envelopes (for example Influenza B, novel influenza) other than Influenza A and avian influenza.

### (Test against bad odor)

The following test uses, as the Example, a "nonwoven fabric in which a radiation generating substance of 10 g/m² is applied on nonwoven fabric of polypropylene fiber (17 g/m²)" that may be used as the second layer 11 of the mask. The test was performed by using ammonia gas, acetic acid gas, trimethylamine gas, isovaleric acid gas, nonenal gas, and indole gas as the subject gas. As for ammonia gas, acetic acid gas, and trimethylamine gas, a detection tube method defined in The Certification Standards of SEK Mark Textile Products, and the nonwoven fabric used in Example that is cut into a 200 cm² piece was used as the sample. On the other hand, for isovaleric acid gas, nonenal gas, and indole gas, the nonwoven fabric used in Example that is cut into a 100 cm² piece was placed in a 5L gas bag, 3L of the subject gas to be measured that is adjusted to a predetermined concentration was injected therein, and the gas concentration 2 hours later was measured by the detection tube. The gas concentration after 2 hours and the reduction rate is depicted in Table 12. As depicted in Table 12, either one of the subject gas demonstrates a large decrease in concentration 2 hours later. Particularly, the isovaleric acid gas, nonenal gas, and indole gas demonstrated a high reduction rate of 90% or more. Therefore, it was found that the mask 1 according to the present embodiment has an effect to reduce various components that serve as a cause of bad odor, such as ammonia gas, acetic acid gas, trimethylamine gas, isovaleric acid gas, nonenal gas, and indole gas.

As described above in details, the mask 1 according to the present embodiment includes: a body portion 2 being air-permeable and being configured to cover at least one portion of a face of a wearer; and a fixing portion 3 configured to fix the body portion 2 against the face of the wearer, and furthermore the body portion 2 has a radiation generating layer that emits radiation to its surroundings, which radiation generates hydroxyl radicals upon reacting to water molecules contained in the air. The hydroxyl radicals generated by the radiation emitted from the radiation generating layer, and hydrogen peroxide further generated from the hydroxyl radicals, inactivates the viruses, bacteria or the like in the surroundings. Therefore, it is possible to inactivate the viruses, bacteria or the like filtered at the body portion 2 or that are present around the body portion. In particular, it is possible to inactivate bacteria including *Staphylococcus aureus, Pseudomonas aeruginosa, Salmonella enteritidis, Escherichia coli* (0-157), and *Moraxella osloensis,* and viruses with envelopes, for example the influenza virus (e.g., Influenza A virus, avian influenza).
Furthermore, the weak radiation generated from the body portion has an effect of stabilizing the immune system of the wearer and improving their immune functions by the so-called hormesis effect. As a result, the ability to resist pollen allergy, infections or the like improve, and by combining this with the inactivation of the above viruses or the like, an extremely large effect can be expected against pollen allergy and infections or the like.
On the other hand, the weak radiation generated from the body portion also has an effect of reducing various components that serve as a cause of bad odor, such as ammonia gas, acetic acid gas, trimethylamine gas, isovaleric acid gas, nonenal gas, and indole gas. Therefore, the displeasure feeling for the wearer of the mask can be reduced in a case of staying in a space with bad odor. Moreover, it is possible to prevent the mask from being contaminated by the bad odor component the wearer themselves give out from inside their mouth.

The present invention is not limited to the above embodiments, and it is a matter of course that various modifications and variations can be made without departing from the features of the present invention.
For example in the present embodiment, the body portion 2 of the mask 1 is of a four layer structure, however this may be three layers or less or five layers or more. Moreover, in the present embodiment there is just one layer of the radiation generating layer that generates radiation, however this may be two layers or more. Moreover, the radiation generating layer may be resin or a rubber member having elasticity, other than the nonwoven fabric. For example, the radiation generating layer is formed by combining the radiation generating substance into resin material followed by molding. Moreover, there is no essential need for the radiation generating layer to cover the entire body portion 2, and may be arranged just on one part of the body portion 2.

Moreover, the present embodiment describes an example that applies the present invention to sanitary masks, but is also applicable to surgical masks and dust-proof masks. For example, with a dust-proof mask, the radiation generating layer is provided in a filter portion.

### REFERENCE SIGNS LIST

- 1: Mask
- 2: Body portion
- 3: Fixing portion
- 10: First layer
- 11: Second layer (Radiation generating layer)
- 12: Third layer
- 13: Fourth layer
- 20: Filtered object

**Table 1: test result of a filtration effect test against particulate matters**

| 1. Test results | | |
|---|---|---|
| Test Items | | Test Results |
| Particle Filtration Efficiency PFE % | 1 | 99.7 |
| | 2 | 99.8 |
| | 3 | 99.5 |
| | 4 | 99.4 |
| | 5 | 99.7 |
| | Average value | 99.6 |

| | | |
|---|---|---|
| *Four layers were stacked from an upstream side in the order of first layer (spunbond 30 g/m²), second layer (special processed nonwoven), third layer (meltblown 22 g/m²), and fourth layer (spunbond 20 g/m²), to use for the test. | | |

### 2. Test method

ASTM F 2299
However, performing no neutralization of particles

### Test conditions

Test area: 49.0 cm²
Test flow rate: 28.3 L/min
Particle size: 0.1 µm (0.100 ± 0.003 µm)
Particle type: JSR SIZE STANDARD PARTICLES SC-0100-D (manufactured by JSR Life Sciences Corporation) Pearl-shaped polystyrene-based standard particles

**Table 2: test result of a filtration effect test against bacteria and viruses**

| 1. BFE | | | | |
|---|---|---|---|---|
| Sample* | Bacterial droplets filtration efficiency (%) | | | |
| | No. 1 | No. 2 | No. 3 | Average value |
| Spunbond 30 g/m²), Special processed nonwoven, Meltblown 22 g/m², and Spunbond 20 g/m² | 99.5 | 99.6 | 99.0 | 99.4 |

### 2. VFE

| Sample* | Bacterial droplets filtration efficiency (%) | | | |
|---|---|---|---|---|
| | No. 1 | No. 2 | No. 3 | Average value |
| Spunbond 30 g/m²), Special processed nonwoven, Meltblown 22 g/m², and Spunbond 20 g/m² | 99.7 | 99.2 | 99.7 | 99.5 |

| | | | | |
|---|---|---|---|---|
| Notes* Four layers were stacked from an upstream side in the order of first layer (spunbond 30 g/m²), second layer (special processed nonwoven), third layer (meltblown 22 g/m²), and fourth layer (spunbond 20 g/m²), to use for the test. | | | | |

### Test method

Bacterial droplets filtration efficiency (BFE) test: ASTM F 2101-14
Test bacteria: Staphylococcus aureus ATCC 6538
Viral droplets filtration efficiency (VFE) test: ASTM F 2101-14, applied accordingly
Test virus: Bacteriophage Phi X174 ATCC 13706-B1
Host bacteria: Escherichia coli E.coli C ATCC 13706

**Table 3: test result of a filtration effect test against pollen**

| 1. Test results | | |
|---|---|---|
| Test Items | | Test Results |
| Filtration Efficiency of pollen particles (%) | 1 | Not less than 99.9 |
| | 2 | 99.9 |
| | 3 | 99.4 |
| | Average value | 99.8 |

| | | |
|---|---|---|
| *Four layers were stacked from an upstream side in the order of first layer (spunbond 30 g/m²), second layer (special processed nonwoven), third layer (meltblown 22 g/m²), and fourth layer (spunbond 20 g/m²), to use for the test. | | |

### 2. Test method

In a state vacuuming the test system at a constant air flow rate, test powder (pollen substitute particles) are dropped at a constant speed from above the filter portion by a particle size selector. The particle mass filtered at the filter portion and the particle mass that had passed through the filter portion were measured, to calculate the filtration efficiency of the pollen particles by the following formula: Filtration Efficiency of pollen particles (%) = Particle mass (mg) filtered at the filter portion / [Particle mass (mg) filtered at the filter portion + Particle mass (mg) passed through the filter portion] x 100

### Test conditions

Test powder (pollen substitute particles): Lycopodium powder (APPIE standard powder)
Test flow rate: 28.3 L/min
Test powder amount: 75 ± 5 mg
Test powder speed: 20 ± 5 mg/min
Temperature and humidity of laboratory: 20 ± 5°, 50 ± 10% RH
[Japan Hygiene Products Industry Association Japan Face Mask Industry Association stipulated test method]

**Table 4: test result of an inactivation test against Influenza A virus (after 30 minutes)**

| 1. Sample type, name, etc. | | | | | | | |
|---|---|---|---|---|---|---|---|
| | Sample No. Sample type, name, etc. | | | | | | |
| | (1) | Nonwoven fabric (Example) | | | | | |

| 2. Control test and Antiviral property test. | | | | | | | |
|---|---|---|---|---|---|---|---|
| Sample No. Preparation etc. | | Control test | | | | Antiviral test | |
| | | Presence of cytotoxicity in washing fluid | Virus addition & collecting test to washing fluid | | | lg(Vc)^{Note 2} | Antiviral activity value - Mv |
| | | | Common Logarithm of collected amount (PFU/mL) | Difference from standard fabric | Decision^{Note 1} | | |
| (1) Original item | | None | 2.85 | 0.0 | Valid | 6.13 | 0.7 |
| -- | | -- | -- | -- | -- | -- | -- |
| Standard fabric (100% cotton, white fabric) | | None | 2.89 | -- | -- | -- | -- |
| | | Common logarithm of virus titer (PFU/test strip) immediately after inoculation: log(Va) | | | | 6.78 | |
| | | Common logarithm of virus titer (PFU/test strip) after leaving for 30 minutes: log(Vb) | | | | 6.52 | |
| | | Reduced value: M (test established condition: reduced value ≤ 1.0) | | | | 0.3 (test established) | |
| Virus titer (PFU/mL) of inoculated virus fluid | | | | | | 3.1E+07 | |

| | | | | | | | |
|---|---|---|---|---|---|---|---|
| Note 1: Decision standard of validity of control test is that there is no cytotoxicity and a difference from the Standard fabric is not more than 0.5 Note 2: Common logarithm of virus titer (PFU/test strip) after leaving antiviral processed fabric for 30 minutes | | | | | | | |

### Test Method: JIS L 1922:2016, applied accordingly (ISO 18184:2014, applied accordingly)

(The time left after inoculating the virus fluid to the test strip was changed from the standard condition of 2 hours to 30 minutes.)

### Measurement method of virus titer: Plaque technique

### Type [host cell] of virus used in test: Influenza A virus (H3N2) ATCC VR-1679 [MDCK cell ATCC CCCL-34]

### Sample (1)

**Table 5: test result of an inactivation test against Influenza A virus (after 2 hours)**

| 1. Sample type, name, etc. | | | | | | | |
|---|---|---|---|---|---|---|---|
| | Sample No. Sample type, name, etc. | | | | | | |
| | (1) | Nonwoven fabric (Example) | | | | | |

| 2. Control test and Antiviral property test. | | | | | | | |
|---|---|---|---|---|---|---|---|
| Sample No. Preparation etc. | | Control test | | | | Antiviral test | |
| | | Presence of cytotoxicity in washing fluid | Virus addition & collecting test to washing fluid | | | lg(Vc)^{Note 2} | Mv^{Note 3} |
| | | | Common Logarithm of collected amount (PFU/mL) | Difference from standard fabric | Decision^{Note 1} | | |
| (1) Original item | | None | 2.55 | 0.2 | Valid | 3.18 | 3.7 |
| -- | | -- | -- | -- | -- | -- | -- |
| Standard fabric (100% cotton, white fabric) | | None | 2.71 | -- | -- | -- | -- |
| | | Common logarithm of virus titer (PFU/test strip) immediately after inoculation: log(Va) | | | | 6.87 | |
| | | Common logarithm of virus titer (PFU/test strip) after leaving for 2 hourse: log(Vb) | | | | 6.58 | |
| | | Reduced value: M (test established condition: reduced value ≤ 1.0^{Note 4}) | | | | 0.3 (test established) | |
| Virus titer (PFU/mL) of inoculated virus fluid | | | | | | 2.9E+07 | |

| | | | | | | | |
|---|---|---|---|---|---|---|---|
| Note 1: Decision standard of validity of control test is that there is no cytotoxicity and a difference from the Standard fabric is not more than 0.5 Note 2: Common logarithm of virus titer (PFU/test strip) after leaving for 2 hours of antiviral processed fabric Note 3: Antiviral activity value (Mv = lg(Va)-lg(Vc)) Calculation formula of antiviral activity value followed SEK mark certification standard specified by Japan Textile Evaluation Technology Council Note 4: The test established condition of reduced value followed SEK mark certification standard specified by Japan Textile Evaluation Technology Council Special notes: Test viral fluid was inoculated from the plane designed by your company Test Method: ISO 18184:2014, applied accordingly Measurement method of virus titer: Plaque technique Type [host cell] of virus used in test: Influenza A virus (H3N2) ATCC VR-1679 [MDCK cell ATCC CCCL-34] Sample (1) | | | | | | | |

**Table 6: test result of an inactivation test against bacteria**

| 1. Staphylococcus aureaus | | | | | |
|---|---|---|---|---|---|
| No. | Sample | | Common logarithm value of viable bacteria (Maximum minimum difference) | | Antibacterial activity value |
| | | | Immediately after inoculation | After 18 hours culturing | |
| (1) | Nonwoven fabric (Example) | Original item | 4.40(0.0) | 1.30(0.0) | 5.8 |
| | -- | -- | -- | -- | -- |
| Comparative sample, (Standard fabric (100% cotton, white fabric)) | | | 4.51(0.0) | 7.06(0.0) | Growth value F: 2.5 |

### 2. Pseudomonas aeruginosa

| No. | Sample | | Common logarithm value of viable bacteria (Maximum minimum difference) | | Antibacterial activity value |
|---|---|---|---|---|---|
| | | | Immediately after inoculation | After 18 hours culturing | |
| (1) | Nonwoven fabric (Example) | Original item | 4.47(0.0) | 1.30(0.0) | 6.0 |
| | -- | -- | -- | -- | -- |
| Comparative sample, (Standard fabric (100% cotton, white fabric)) | | | 4.49(0.0) | 7.34(0.0) | Growth value F: 2.8 |

### 3. Escherichia coli 0157:H7

| No. | Sample | | Common logarithm value of viable bacteria (Maximum minimum difference) | | Antibacterial activity value |
|---|---|---|---|---|---|
| | | | Immediately after inoculation | After 18 hours culturing | |
| (1) | Nonwoven fabric (Example) | Original item | 4.48(0.0) | 2.45(1.0) | 5.0 |
| | -- | -- | -- | -- | -- |
| Comparative sample, (Standard fabric (100% cotton, white fabric)) | | | 4.49(0.0) | 7.44(0.2) | Growth value F: 2.9 |

| | | | | | |
|---|---|---|---|---|---|
| Note: Used a test bacterial suspension added with a surfactant (Tween 80) Test method: JIS L1902:2015, Bacterial fluid absorption method Test bacteria type: Staphylococcus aureaus NBRC 12732 Pseudomonas aeruginosa NBRC 3080 Escherichia coli 0157: H'7 ATCC 43888 Sample (1) | | | | | |

**Table 7: test result of an inactivation test against bacteria 1. Moraxella osloensis**

| No. | Sample | | Common logarithm value of viable bacteria (Maximum minimum difference) | | Antibacterial activity value |
|---|---|---|---|---|---|
| | | | Immediately after inoculation | After 18 hours culturing | |
| (1) Nonwoven fabric (Example) | | Original item | 3.99(0.1) | 1.30(0.0) | 6.0 |
| -- | | -- | -- | -- | -- |
| Comparative sample, (Standard fabric (100% cotton, white fabric)) | | | 4.46(0.1) | 7.33(0.1) | Growth value F: 2.5 |

### 2. Salmonella enteritidis

| No. | Sample | | Common logarithm value of viable bacteria (Maximum minimum difference) | | Antibacterial activity value |
|---|---|---|---|---|---|
| | | | Immediately after inoculation | After 18 hours culturing | |
| (1) Nonwoven fabric (Example) | | Original item | 4.40(0.1) | 4.09(0.2) | 3.3 |
| -- | | -- | -- | -- | -- |
| Comparative sample, (Standard fabric (100% cotton, white fabric)) | | | 4.50(0.1) | 7.37(0.1) | Growth value F: 2.5 |

| | | | | | |
|---|---|---|---|---|---|
| Note: Used a test bacterial suspension added with a surfactant (Tween 80) Test method: JIS L1902:2015, Bacterial fluid absorption method Test bacteria type: Moraxella osloensis ATCC 19976 Salmonella enteritidis NBRC 3313 Sample (1) | | | | | |

**Table 8: test result of an inactivation test against bacteria - viable bacteria count for each test strip**

| 1. Moraxella osloensis | | | | | |
|---|---|---|---|---|---|
| No. | Sample | | Viable bacteria count (CFU/test strip) | | |
| | | | No.1 | No.2 | No.3 |
| (1) | Nonwoven fabric (Example) | Immediately after inoculation | 1.1E+04 | 8.2E+03 | 1.0E+04 |
| | | After 18 hours culturing | 2.0E+01 | 2.0E+01 | 2.0E+01 |
| | -- | -- | -- | -- | -- |
| Comparative sample, (Standard fabric (100% cotton, white fabric)) | | Immediately after inoculation | 2.7E+04 | 3.2E+03 | 2.8E+04 |
| | | After 18 hours culturing | 2.3E+07 | 2.0E+07 | 2.2E+07 |

### 2. Salmonella enteridis

| No. | Sample | | Viable bacteria count (CFU/test strip) | | |
|---|---|---|---|---|---|
| | | | No.1 | No.2 | No.3 |
| (1) | Nonwoven fabric (Example) | Immediately after inoculation | 2.2E+04 | 2.8E+04 | 2.6E+04 |
| | | After 18 hours culturing | 1.7E+04 | 9.8E+03 | 1.1E+04 |
| -- | | -- | -- | -- | -- |
| Comparative sample, (Standard fabric (100% cotton, white fabric)) | | Immediately after inoculation | 3.2E+04 | 3.3E+04 | 2.9E+04 |
| | | After 18 hours culturing | 2.5E+07 | 2.1E+07 | 2.4E+07 |

**Table 9: theoretical reduction rate of viruses/bacteria**

| Influenza A virus | | |
|---|---|---|
| Number of viruses immediately after inoculation | 6,025,596 | Reduction rate (%) |
| After 30 mines elapse | 1,348,963 | 77.61 |

| | | |
|---|---|---|
| Number of viruses immediately after inoculation | 7,413,102 | Reduction rate (%) |
| After 2 hours elapse | 1,514 | 99.98 |

### Staphylococcus aureus

| | | |
|---|---|---|
| Number of bacteria immediately after inoculation | 32,359 | Reduction rate (%) |
| After 18 hours elapse | 20 | 99.94 |

### Pseudomonas aeruginosa

| | | |
|---|---|---|
| Number of bacteria immediately after inoculation | 30,903 | Reduction rate (%) |
| After 18 hours elapse | 20 | 99.94 |

### Escherichia coli 0157

| | | |
|---|---|---|
| Number of bacteria immediately after inoculation | 30,903 | Reduction rate (%) |
| After 18 hours elapse | 282 | 99.09 |

### Moraxella osloensis

| | | |
|---|---|---|
| Number of bacteria immediately after inoculation | 28,840 | Reduction rate (%) |
| After 18 hours elapse | 20 | 99.93 |

### Salmonella enteritidis

| | | |
|---|---|---|
| Number of bacteria immediately after inoculation | 31,623 | Reduction rate (%) |
| After 18 hours elapse | 12,303 | 61.10 |

**Table 10: preliminary test of an inactivation test against avian influenza**

| Test object | Remaining virus titer after 30 minutes (log₁₀EID₅₀/0.2 mL) |
|---|---|
| Example 1 | 6.5 |
| Example 2 | 5.5 |
| Comparative Example 1 | 6.25 |
| Test virus | 7.25 |

**Table 11: final test of the inactivation test against avian influenza - residual virus titer (log₁₀EID₅₀/0.2 mL)**

| Time left | | Example 1 | Example 2 | Example 3 | Comparative Example | Test virus |
|---|---|---|---|---|---|---|
| 10 mins | 1st | 3.5 | 1.75 | 6.75 | 6.75 | 7.5 |
| | 2nd | 5.25 | 2.5 | 6.5 | 6.5 | 7.25 |
| 30 mins | 1st | 5.0 | 1.5 | --- | 7.0 | 7.5 |
| | 2nd | 5.5 | 0.75 | 6.5 | 6.75 | 6.75 |
| 1 hours | 1st | 4.25 | --- | 6.5 | 7.25 | 6.75 |
| 2 hours | 1st | 2.0 | ≤1.5 | 6.75 | 6.5 | 7.25 |
| | 2nd | ≤0.5 | 0.66 | 5.75 | 6.75 | 7.5 |

**Table 12: reductions rates of gases that serve as bad odor components**

| Ammonia gas | | | |
|---|---|---|---|
| Sample | Initial concentration (ppm) | 2 hours later | |
| | | Gas concentration (ppm) | Reduction rate (%) |
| Example | 100 | 8.3 | 89 |
| Blank (blank test) | 100 | 20 | --- |

### Acetic acid gas

| Sample | Initial concentration (ppm) | 2 hours later | |
|---|---|---|---|
| | | Gas concentration (ppm) Reduction rate (%) | |
| Example | 30 | 5.2 | 74 |
| Blank (blank test) | 30 | 20 | --- |

### Trimethylamine gas

| Sample | Initial concentration (ppm) | 2 hours later | |
|---|---|---|---|
| | | Gas concentration (ppm) | Reduction rate (%) |
| Example | 28 | 6.1 | 76 |
| Blank (blank test) | 28 | 25 | --- |

### Isovaleric gas

| Sample | Reduction rate of 2 hours later (%) |
|---|---|
| Example | 95 |

### Nonenal gas

| Sample | Reduction rate of 2 hours later (%) |
|---|---|
| Example | 95 |

### Indole gas

| Sample | Reduction rate of 2 hours later (%) |
|---|---|
| Example | 98 |

## Claims

1. A mask (1), comprising:
a body portion (2) being air-permeable and being configured to cover at least one portion of a face of a wearer; and a fixing portion (3) coupled to the body portion (2), configured to fix the body portion (2) against the face of the wearer,
wherein the body portion (2) has a radiation generating layer (11) configured to generate radiation to its surroundings, the radiation generating hydroxyl radicals upon reacting to water molecules contained in the air, and
wherein the radiation generating layer (11) is a nonwoven fabric having a radiation generating substance applied on at least one plane thereof, the radiation generating substance generating the radiation to its surroundings,
**characterized in that**
a mass per unit area of the nonwoven fabric is not more than 17 g/m²,
an application density of the radiation generating substance is 10 g/m² or more, and
a mass per unit area of the nonwoven fabric in a state in which the radiation generating substance is applied thereon is not more than 32 g/m².

2. A mask (1) according to claim 1, wherein the mass per unit area of the nonwoven fabric in the state in which the radiation generating substance is applied thereon is not more than 27 g/m²

3. A mask (1) according to claim 1or 2, wherein the nonwoven fabric is made of polypropylene.

4. A mask (1) according to any one of claims 1 to 3, wherein the body portion (2) has a laminate structure including a lamination of the radiation generating layer (11) and a nonwoven fabric separate from the radiation generating layer (11).

## Patentansprüche

1. Eine Maske (1), umfassend:
einen Körperabschnitt (2), der luftdurchlässig ist und konfiguriert ist, mindestens einen Teil eines Gesichts eines Trägers zu bedecken; und einen Befestigungsabschnitt (3), der mit dem Körperabschnitt (2) gekoppelt ist und konfiguriert ist, den Körperabschnitt (2) an dem Gesicht des Trägers zu befestigen, wobei
der Körperabschnitt (2) eine strahlungserzeugende Schicht (11) aufweist, die konfiguriert ist, Strahlung an ihre Umgebung abzugeben, wobei die Strahlung bei Reaktion mit in der Luft enthaltenen Wassermolekülen Hydroxylradikale erzeugt, und wobei
die strahlungserzeugende Schicht (11) ein Vliesstoff mit einer strahlungserzeugenden Substanz ist, die auf mindestens einer Ebene davon aufgebracht ist, wobei die strahlungserzeugende Substanz die Strahlung an ihre Umgebung erzeugt,
**gekennzeichnet dadurch, dass**
die Flächenmasse des Vliesstoffs nicht mehr als 17 g/m2 beträgt,
eine Auftragsdichte der strahlungserzeugenden Substanz 10 g/m2 oder mehr beträgt und
eine Masse pro Flächeneinheit des Vliesstoffs in einem Zustand, in dem die strahlungserzeugende Substanz darauf aufgebracht ist, nicht mehr als 32 g/m2 beträgt.

2. Die Maske (1) gemäß Anspruch 1, wobei die Masse pro Flächeneinheit des Vliesstoffs in dem Zustand, in dem die strahlungserzeugende Substanz darauf aufgebracht ist, nicht mehr als 27 g/m2 beträgt.

3. Die Maske (1) gemäß Anspruch 1 oder 2, wobei der Vliesstoff aus Polypropylen hergestellt ist.

4. Die Maske (1) gemäß einem der Ansprüche 1 bis 3, wobei der Körperabschnitt (2) eine Laminatstruktur aufweist, die eine Laminierung der strahlungserzeugenden Schicht (11) und eines von der strahlungserzeugenden Schicht (11) getrennten Vliesstoffs umfasst.

## Revendications

1. Masque (1), comprenant :
une portion de corps (2) perméable à l'air et configurée pour recouvrir au moins une portion du visage d'un porteur ; et
une portion de fixation (3) couplée à la portion de corps (2), configurée pour fixer la portion de corps (2) contre le visage du porteur,
dans lequel la portion de corps (2) comporte une couche générant un rayonnement (11) configurée pour générer un rayonnement vers son environnement, le rayonnement générant des radicaux hydroxyles lors d'une réaction avec les molécules d'eau contenues dans l'air, et
dans lequel la couche générant un rayonnement (11) est un non-tissé sur au moins un plan duquel est appliquée une substance générant un rayonnement, la substance générant un rayonnement générant le rayonnement vers son environnement,
**caractérisé en ce que**
la masse par unité de surface du non-tissé n'est pas supérieure à 17 g/m²,
la densité d'application de la substance générant un rayonnement est supérieure ou égale à 10 g/m², et
la masse par unité de surface du non-tissé dans un état où y est appliquée la substance générant un rayonnement n'est pas supérieure à 32 g/m².

2. Masque (1) selon la revendication 1, dans lequel la masse par unité de surface du non-tissé dans l'état où y est appliquée la substance générant un rayonnement n'est pas supérieure à 27 g/m².

3. Masque (1) selon la revendication 1 ou 2, dans lequel le non-tissé est constitué de polypropylène.

4. Masque (1) selon l'une quelconque des revendications 1 à 3, dans lequel la portion de corps (2) présente une structure stratifiée comprenant une stratification de la couche générant un rayonnement (11) et d'un non-tissé séparé de la couche générant un rayonnement (11).
